# FASCICULE DE BREVET EUROPEEN

(11) **EP 0 693 060 B1**
(45) Date de publication et mention de la délivrance du brevet: **18.03.1998**
(21) Numéro de dépôt: 94913146.0
(22) Date de dépôt: 07.04.1994
(51) Int. Cl.: C07D 251/54

(54) **PROCEDE DE PREPARATION DE FLUORO-6 HALOGENO-2 QUINOLEINE**
VERFAHREN ZUR HERSTELLUNG VON 6-FLUORO-2-HALOGENO-CHINOLIN
METHOD OF PREPARATION OF 6-FLUORO-2-HALOGEN QUINOLINE

(30) Priorité: 08.04.1993 FR 9304163
(43) Date de publication de la demande: 24.01.1996
(73) Titulaire: RHONE-POULENC RORER S.A., 92160 Antony (FR)
(72) Inventeur: GARCIA, Hervé, F-69360 Communay (FR); JACQUOT, Roland, F-69110 Sainte-Foy-les-Lyon (FR); LEON, Patrick, F-69160 Tassin-la-Demi-Lune (FR)
(74) Mandataire: Savina, Jacques
(86) Numéro de dépôt international: FR9400391
(87) Numéro de publication internationale: WO9424113

(56) Documents cités:
- EP-A- 0 148 623
- EP-A- 0 236 140
- EP-A- 0 379 412
- WO-A-93/07127
- FR-A- 2 225 166
- Tetrahedron Letters 1982, 1099-1103

## Description

La présente invention concerne la préparation de fluoro-6 halogéno-2 quinoléine de formule générale : 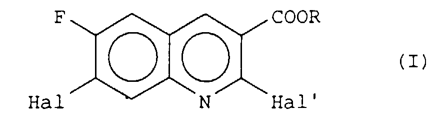 dans laquelle R est un atome d'hydrogène ou un radical alcoyle, Hal et Hal' sont des atomes d'halogène identiques ou différents.

Dans Methoden den Organischen Chemie, Band E7a, Houben Weyl pages 516, 517, 548, 549 et 687 ont été citées des réactions d'halogénation de quinolones ou d'isoquinolones.

Dans le brevet US 4 970 213 ont été décrits des acides fluoro-6 chloro-2 quinoléine carboxyliques de structure : 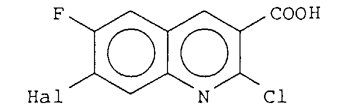 dans laquelle Hal est un atome de fluor ou de chlore, utiles comme intermédiaires pour la préparation de benzonaphtyridines-1,8 ayant une activité antimicrobienne.

Le procédé selon la présente invention est également utile pour la préparation de dérivés de la benzonaphtyridine-1,8 antimicrobiens, de plus il permet d'opérer dans des conditions douces et d'obtenir des rendements améliorés et évite de passer intermédiairement par des produits instables.

Dans la formule générale (I) lorsque R représente un radical alcoyle, ce dernier est droit ou ramifié et contient 1 à 4 atomes de carbone; le symbole Hal est avantageusement choisi parmi le chlore ou le fluor et le symbole Hal' est choisi parmi le chlore ou le brome.

Selon la présente invention le dérivé de la quinoléine de formule générale (I) peut être préparé par action d'un agent d'halogénation sur l'hydroxy-1 quinolone de formule générale : 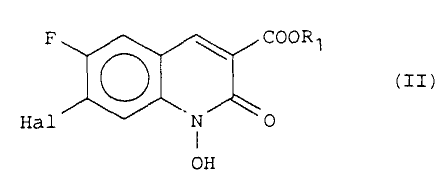 dans laquelle Hal est défini comme précédemment et R₁ est défini comme R à l'exception de représenter un atome d'hydrogène, suivie éventuellement de la libération de la fonction acide si l'on veut obtenir un dérivé de la quinoléine de formule générale (I) pour lequel R est un atome d'hydrogène.

La réaction s'effectue dans un solvant organique comme par exemple un solvant halogéné (particulièrement un solvant chloré comme le dichlorométhane, le dichloro-1,2 éthane, le trichloro-1,1,1 éthane, le chlorobenzène ou le dichlorobenzène notamment) ou dans un solvant aromatique (par exemple le toluène, le nitrobenzène, le diphényléther...) à une température comprise entre 20°C et la température de reflux du mélange réactionnel.

Lorsque l'on veut obtenir la chloro-2 quinoléine l'agent d'halogénation peut être choisi parmi le trichlorure de phosphore, le pentachlorure de phosphore, le chlorure de thionyle, le chlorure de sulfuryle, le dichlorosulfure, le chlorure stanneux, le chlorure cuivreux, le trichlorure de titane, le chlorure ferreux, le chlorure de chrome^{II}, l'hydrochloro triphénylphosphorane, le dichloro triphénylphosphorane ou le chlore. Lorsque l'on veut obtenir la bromo-2 quinoléine l'agent d'halogénation peut être choisi parmi le tribromure de phosphore, l'hydrobromo triphénylphosphorane ou le dibromo triphénylphosphorane.

Dans les cas où l'on a obtenu l'ester et où l'on veut obtenir l'acide de formule générale (I) pour lequel R est un atome d'hydrogène, l'hydrolyse de l'ester peut être mise en oeuvre par toute méthode connue pour obtenir un acide à partir d'un ester sans toucher au reste de la molécule. Notamment on effectue l'hydrolyse en milieu acide, par exemple en présence d'acide chlorhydrique, d'acide sulfurique ou méthanesulfonique. Elle peut aussi être effectuée en milieu hydroalcoolique basique (soude, potasse par exemple).

L'hydroxy-1 quinolone de formule générale (II) peut être obtenue par cyclisation d'un dérivé nitré de formule générale : 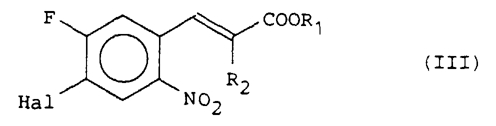 dans laquelle Hal et R₁ sont définis comme précédemment, et R₂ est défini comme R₁ ou représente un radical carbamoyle ou cyano, par hydrogénation catalytique en milieu acide.

L'hydrogénation catalytique s'effectue en présence de palladium sur charbon ou de platine, à une température comprise entre 0 et 130°C, en présence d'un acide organique ou minéral qui n'altère pas le reste de la molécule. A titre d'exemple on opère dans l'acide acétique ou dans l'acide formique, il est également possible d'opérer au moyen d'acide chlorhydrique dilué ou d'acide sulfurique dilué en milieu hydro-alcoolique. La réaction est mise en oeuvre jusqu'à ce que la consommation d'hydrogène diminue brusquement. De préférence on opère sous pression atmosphérique.

Le dérivé nitré de formule générale (III) peut être préparé par action d'un dérivé de l'acide malonique de formule générale :

R₁OCO-CH₂-R₂ (IV)

dans laquelle R₁ et R₂ sont définis comme précédemment, sur un dérivé du nitrobenzaldéhyde de formule générale : 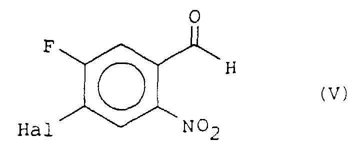 dans laquelle Hal est défini comme précédemment.

La réaction s'effectue généralement en milieu basique [par exemple en présence d'un bicarbonate alcalin (bicarbonate de sodium), d'un hydrure (hydrure de sodium) ou d'un alcoolate à une température comprise entre 0 et 150°C, dans un solvant organique tel qu'un anhydride (anhydride acétique par exemple) ou tel qu'un amide (diméthyl-formamide, N-méthylpyrrolidone par exemple) en opérant en présence de tamis moléculaires ou de tout autre agent desséchant ou encore dans un mélange de solvants comme un mélange solvant aprotique polaire/anhydride acétique (diméthylformamide/anhydride acétique, N-méthyl pyrrolidone/anhydride acétique par exemple). Il est également possible d'opérer en milieu biphasique. Il n'est pas indispensable d'isoler le produit de formule générale (III) pour le mettre en oeuvre dans la réaction suivante.

Le fluoronitrobenzaldéhyde de formule générale (V) est obtenu par nitration du fluorobenzaldéhyde de formule générale : 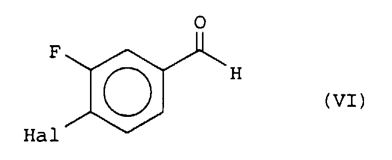 dans laquelle Hal est défini comme précédemment.

la réaction s'effectue avantageusement par l'acide nitrique concentré sous forme d'un mélange sulfonitrique, ou d'un mélange acide nitrique/acide acétique, à une température comprise entre 0 et 90°C.

Le chloro-4 fluoro-3 benzaldéhyde peut être préparé selon la méthode décrite dans la demande européenne EP 289 942.

Selon l'invention la préparation de la fluoro-6 halogéno-2 quinoléine de formule générale (I) est utile pour la synthèse de dérivés de la benzo[b]naphtyridine-1,8 de formule générale : 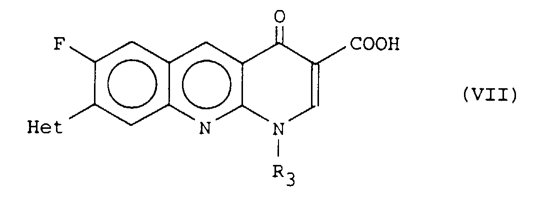 dans laquelle soit R₃ (qui représente un radical alcoyle, fluoroalcoyle, cycloalcoyle contenant 3 à 6 atomes de carbone, alcoyloxy ou alcoylamino) et Het (qui est un radical hétérocyclyle azoté), sont tels que définis pour les substituants en positions -1 et -8, dans la demande européenne EP 431 991 et le brevet US 5 004 745, soit R₃ est un atome d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino, et Het est un radical azétidinyle-1 substitué [en position -3 par un radical R₄ qui peut être un atome d'hydrogène ou un radical hydroxy, amino, alcoylamino dont la partie alcoyle est éventuellement substituée par un radical amino ou hydroxy ou peut représenter un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou peut représenter un radical cycloalcoylamino contenant 3 à 6 chaînons, ou un radical alcanoylamino, N-alcoyl N-alcanoyl amino ou aminoalcoylphénylamino, et substitué en positions -2 et -3 par des radicaux R₅ et R₆ identiques ou différents qui représentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien disubstitué en position -2 par des radicaux R₅ et R₆ qui représentent des radicaux alcoyle], étant entendu que les radicaux alcoyle et alcanoyle cités ci-dessus sont droits ou ramifiés et contiennent 1 à 4 atomes de carbone.

Les benzo[b]naphtyridines-1,8 de formule générale (VII) peuvent être obtenues par le procédé selon l'invention en opérant de la manière suivante :

Le dérivé de la benzonaphtyridine de formule générale (VII) peut être obtenu à partir de l'acide chloro (ou bromo)-2 fluoro-6 quinoléine carboxylique de formule générale (I) dans laquelle R est un atome d'hydrogène, selon ou par analogie avec la méthode décrite dans les demandes EP 431 991 ou WO 93/07144, ou les brevets US 5 004 745 ou US 4 970 213.

Le dérivé de la benzonaphtyridine de formule générale (VII) peut également être obtenu à partir de l'ester de formule générale (I) pour lequel R est un radical alcoyle, en opérant comme suit :

Une amine de formule générale :

R₃-NH-CH₂-CH₂-R₇ (VIII)

dans laquelle R₃ est défini comme précédemment et R₇ est un radical alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, (les radicaux alcoyle étant droits ou ramifiés et contenant 1 à 4 atomes de carbone), est condensée sur l'halogéno fluoro quinoléine de formule générale (I) dans laquelle R est un radical alcoyle, de manière à obtenir un fluoro ester de formule générale : 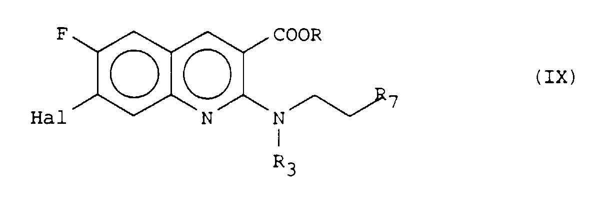 dans laquelle Hal, R₃ et R₇ sont définis comme précédemment et R représente un radical alcoyle.

La condensation s'effectue en milieu basique dans un solvant organique tel qu'un hydrocarbure aromatique (toluène par exemple), un amide (diméthylformamide, N-méthyl pyrrolidone par exemple), un éther (tétrahydrofuranne par exemple), un sulfoxyde (diméthylsulfoxyde par exemple), un solvant chloré (dichlorométhane, dichloroéthane, chlorobenzène par exemple) ou un alcool à une température comprise entre -10 et 120°C.

A titre d'exemple les bases utilisées peuvent être choisies parmi les carbonates alcalins (carbonate de sodium ou de potassium), les alcoolates ou un hydrure alcalin (hydrure de sodium).

Il est entendu que, dans l'alternative où le symbole R₃ représente un radical carboxyalcoyle, ce dernier est protégé préalablement à la réaction. L'élimination du radical protecteur est effectuée de préférence après la réaction d'oxydation, sur le dérivé de la benzonaphtyridine de formule générale (XI) décrit ci-après. La protection et la libération de la fonction acide s'effectuent selon les méthodes habituelles qui n'altèrent pas le reste de la molécule. Notamment selon les méthodes qui ont été citées précédemment.

La fluoro quinoléine de formule générale (IX) est cyclisée en milieu basique pour préparer la tétrahydro-1,2,3,4 benzo[b]naphtyridine-1,8 de formule générale : 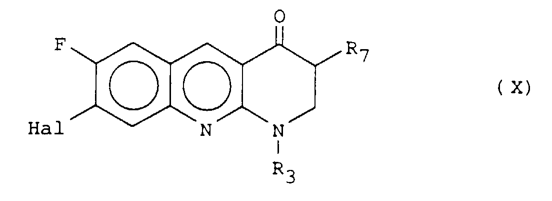 dans laquelle Hal, R₃ et R₇ sont définis comme précédemment.

La réaction s'effectue à une température comprise entre -70 et 120°C en présence d'une base comme un alcoolate (éthylate de sodium, méthylate de sodium, t.butylate de potassium par exemple), un hydrure alcalin (hydrure de sodium par exemple), ou encore un hydroxyde alcalin en opérant par transfert de phase. On opère avantageusement dans un solvant aprotique polaire (par exemple diméthylformamide, tétrahydrofuranne) ou dans un alcool (éthanol, méthanol par exemple) dans un glyme ou dans un glycol (éthylène glycol par exemple). Lorsque l'on effectue la réaction par transfert de phase, on opère avantageusement dans un solvant chloré comme le chlorure de méthylène, la base étant en solution dans la phase aqueuse.

La tétrahydro-1,2,3,4 benzo[b]naphtyridine-1,8 de formule générale (X) est oxydée pour préparer la benzo[b]naphtyridine-1,8 de formule générale : 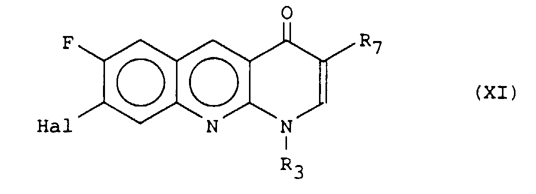 dans laquelle Hal, R₃ et R₇ sont définis comme précédemment.

L'oxydation s'effectue par l'eau oxygénée, éventuellement en présence d'iodure de potassium, dans un solvant organique tel qu'un alcool (éthanol par exemple), à une température comprise entre 0 et 120°C. Il est également possible d'opérer en milieu biphasique dans un mélange eau/solvant chloré (dichlorométhane, dichloroéthane...).

L'hétérocycle Het est condensé sur la benzo[b]naphtyridine-1,8 de formule générale (XI) ou l'acide correspondant pour préparer un dérivé de la benzonaphtyridine de formule générale (VII), en opérant selon ou par analogie avec les méthodes décrites dans la demande européenne EP 431 991 et le brevet US 5 004 745 puis le cas échéant par transformation de l'ester, de l'amide ou du nitrile obtenu en un acide de formule générale (VII). Les dérivés de la benzonaphtyridine de formule générale (VII) sont des antimicrobiens dont les activités ont été décrites dans la demande européenne et le brevet américain cités ci-dessus. Les dérivés de la benzonaphtyridine de formule générale (VII) pour lesquels Het est un radical azétidinyle, qui sont définis plus en détail dans la demande internationale WO 93/07144, présentent également des propriétés antibactériennes. Ils manifestent une activité remarquable in vitro et in vivo sur les germes gram positifs et aussi sur les germes gram négatifs. In vitro, ils sont actifs à une concentration comprise entre 0,06 et 4 µg/cm³ sur staphylococcus aureus IP 8203 et à une concentration comprise entre 0,25 et 20 µg/cm³ sur Escherichia coli souche NIHJ JC2. In vivo, ils sont actifs sur les infections expérimentales de la souris à staphylococcus aureus IP 8203 à des doses comprises entre 10 et 200 mg/kg par voie orale.

Les produits issus du procédé selon la présente invention, ainsi que les produits auquels ils conduisent, peuvent être éventuellement purifiés par des méthodes physiques telles que la cristallisation ou la chromatographie.

Les exemples suivants donnés à titre non limitatif illustrent la présente invention.

### Exemple 1

15,0 g d'éthoxycarbonyl-3 difluoro-6,7 hydroxy-1 oxo-2 quinoléine (titre 90 % - 50,1 mmoles) [contenant 10 % molaire d'éthoxycarbonyl-3 difluoro-6,7 oxo-2 quinoléine] et 100 cm³ de dichloro-1,2 éthane sont placés sous atmosphère d'argon puis additionnés de 22 cm³ de trichlorure de phosphore (251 mmoles) et portés à reflux. Après agitation du mélange réactionnel pendant 3 heures 30 minutes, on ajoute 5,5 cm³ de chlorure de phosphoryle (59 mmoles) et chauffe à reflux à nouveau pendant 3 heures 30 minutes. Le mélange réactionnel est versé sur 300 g de glace et agité pendant 15 minutes. Après décantation, la phase aqueuse est extraite par 2 fois 50 cm³ de dichloro-1,2 éthane, les phases organiques sont rassemblées, lavées par 200 cm3 d'une solution saturée de bicarbonate de sodium, puis 150 cm³ d'eau et enfin par 150 cm³ d'une solution saturée de chlorure de sodium. Après séchage et concentration à sec, on obtient 13,4 g de chloro-2 éthoxycarbonyl-3 difluoro-6,7 quinoléine sous forme d'un solide de couleur crème. (Titre HPLC : >99 % ; R % = 88 %).

Le produit obtenu peut être purifié par recristallisation : Une portion du solide obtenu : 7,0 g, est dissoute à 90°C dans 157 cm3 d'un mélange d'éthanol à 95 % et d'eau (60/40 en volumes). On laisse refroidir à température ambiante, sous agitation. Le précipité est lavé par 10 cm³ d'eau glacée, par 10 cm³ d'un mélange eau/éthanol (4/1 en volumes), puis par 3 fois 25 cm³ d'eau glacée et séché sous pression réduite sur P₂O₅. On obtient ainsi 6,58 g de chloro-2 éthoxycarbonyl-3 difluoro-6,7 quinoléïne sous forme d'un solide blanc fondant à 111-112°C (capillaire). (Titre HPLC : 100 % ; R % = 94 %).

L'éthoxycarbonyl-3 difluoro-6,7 hydroxy-1 oxo-2 quinoléine peut être préparée de la manière suivante :

Dans un réacteur de 40 cm³, on introduit 130 g d'acide acétique et 1 g de palladium sur charbon à 5% de palladium. Le réacteur est purgé par un courant d'azote, puis sous agitation et sous pression atmosphérique, on fait passer un courant d'hydrogène (3 litres/heure) en chauffant à 55°C. Lorsque la température de 55°C est atteinte, on ajoute, en 35 minutes, 128,5 g de la solution à 21,5 % de (difluoro-3,4 nitro-6 benzylidène) malonate d'éthyle. La température est maintenue à 55°C pendant toute la durée de l'injection. 5 minutes apres la fin de l'addition la consommation d'hydrogène diminue brusquement. L'appareil est purgé par un courant d'azote pendant 10 minutes. Le catalyseur est filtré. On ajoute au filtrat en 10 minutes, 300 g d'eau permutée. La suspension obtenue est agitée pendant 1 heure à 20°C puis filtrée et lavée par 3 fois 50 cm³ d'eau permutée. Le gâteau est séché à l'étuve à 40°C sous pression réduite (13,3 kPa) pendant 4 heures. On obtient ainsi 17,7 g d'éthoxycarbonyl-3 difluoro-6,7 hydroxy-1 oxo-2 quinoléine à 90 % de pureté fondant à 215-218°C avec décomposition (comprenant 10 % d'éthoxycarbonyl-3 difluoro-6,7 oxo-2 quinoléine).

Le (difluoro-3,4 nitro-6 benzylidène) malonate d'éthyle peut être préparé de la manière suivante :
31,82 g de difluoro-3,4 nitro-6 benzaldéhyde (titre 90 % ; 153 mmoles) sont placés sous argon à 20°C avec 32,80 g de malonate d'éthyle (204 mmoles) et 60 cm³ d'anhydride acétique. On ajoute, sous agitation, 28,56 g de bicarbonate de sodium (340 mmoles). La suspension est maintenue 2 heure à environ 20°C, puis le mélange hétérogène orangé est chauffé 3 heures à une température d'environ 70°C. On refroidit à 52°C puis on verse 60 cm³ d'acide acétique, puis à température ambiante (20°C) 30 cm³ d'eau. On laisse agiter encore une nuit à cette température. On obtient ainsi 234,4 g d'une solution orange foncé de (difluoro-3,4 nitro-6 benzylidène) malonate d'éthyle à 21,5 % (poids) qui utilisée telle quelle dans l'étape suivante.

Le difluoro-3,4 nitro-6 benzaldéhyde est préparé de la manière_suivante :
A 520 cm³ d'acide sulfurique, sous agitation, refroidi, à 0°C, on ajoute, en 30 minutes, 60 cm³ d'acide nitrique fumant. A la solution obtenue, on ajoute, en 30 minutes, à environ 0°C, 100 g de difluoro-3,4 benzaldéhyde. On laisse remonter la température à environ 20°C et agite encore 3 heures à cette température. Après refroidissement à environ 5°C, le mélange réactionnel est versé, en 30 minutes, sous forte agitation, dans 1200 g de glace pilée. On laisse la température remonter à environ 20°C et extrait par 2 fois 600 cm³ de toluène. Les extraits organiques réunis sont lavés par 3 fois 1 000 cm³ d'eau, et concentrées sous pression réduite (20 kPa) à 50°C. On obtient 113 g de difluoro-3,4 nitro-6 benzaldéhyde sous forme d'une huile brune qui est utilisée telle quelle dans les synthèses ultérieures. Un échantillon purifié de difluoro-3,4 nitro-6 benzaldéhyde donne les caractéristiques suivantes :
PE(6,66 Pa) = 46°C.
Spectre de RMN (400 MHz, DMSO, T = 298°K)
10,20 ppm (1H, 1s) ; 8,5 ppm (1H, 1q) ; 8,05 ppm (1H, 1q).

### Exemple 2

Un mélange d'éthoxycarbonyl-3 difluoro-6,7 hydroxy-1 oxo-2 quinoléine (25 g à 75 % molaire soit 69,65 mmoles), d'éthoxycarbonyl-3 difluoro-6,7 oxo-2 quinoléine (25 g à 5 % molaire, soit 4,9 mmoles), de trichlorure de phosphore (59,6 g soit 433,5 mmoles) et de chlorure de phosphoryle (15,05 g soit 98,05 mmoles) dans le dichloro-1,2 éthane (170 cm³) est chauffé au reflux pendant 3 heures. Après refroidissement à 0°C, on verse le mélange réactionnel sur de la glace fondante (250 g de glace + 250 g d'eau à 0°C) et laisse décanter pendant la nuit. La phase organique est lavée par une solution de NaHCO₃ saturée (100 cm³), puis par de l'eau (100 cm³) puis concentrée à sec pour obtenir 19,8 g d'éthoxycarbonyl-3 difluoro-6,7 chloro-2 quinoléine de titre 90 % (CLHP), soit un rendement de 88 %.

L'éthoxycarbonyl-3 difluoro-6,7 chloro-2 quinoléine brute [7,0 g à 90 % (titre HPLC)] est recristallisée dans 157 cm³ de mélange éthanol/eau (60/40 en volumes) pour donner 5,5 g d'éthoxycarbonyl-3 difluoro-6,7 chloro-2 quinoléine pure, soit un rendement de recristallisation de 88 %.

### Exemple 3

En opérant comme à l'exemple 2, mais dans 170 cm³ de toluène et en chauffant à reflux pendant 4 heures 15 minutes, on obtient 21,8 g d'éthoxycarbonyl-3 difluoro-6,7 chloro-2 quinoléine brute de titre 85 %, soit un rendement de 92 %.

Les produits obtenus par le procédé selon l'invention peuvent être utilisés de la manière suivante :

### Exemple d'utilisation 1

La chloro-2 éthoxycarbonyl-3 difluoro-6,7 quinoléine est convertie en acide chloro-2 difluoro-6,7 quinoléine carboxylique-3 selon les méthodes habituelles et peut conduire ainsi aux dérivés de la benzo[b]naphtyridine-1,8 décrits dans le brevet US 4 970 213.

### Exemple d'utilisation 2

### Préparation de l'éthoxycarbonyl-3 difluoro-6,7 (N-méthyl N-β éthoxycarbonyléthyl) amino-2 quinoléine :

A une solution de 72 g de chloro-2 éthoxycarbonyl-3 difluoro-6,7 quinoléïne préparée comme décrit à l'exemple 1 et 45,1 g de N-méthyl, N-β éthoxycarbonyléthyl amine dans 750 cm³ de toluène, on ajoute 56,2 g de carbonate de sodium. La suspension obtenue est chauffée à environ 90°C puis agitée 4 heures à cette température. Le mélange réactionnel est ensuite refroidi à environ 20°C puis lavé par 3 fois 400 cm³ d'eau. La phase organique est concentrée à sec sous pression réduite (20kPa) à environ 50°C. On obtient 94 g d'éthoxycarbonyl-3 difluoro-6,7 (N-méthyl N-β éthoxycarbonyléthyl) amino-2 quinoléïne sous forme d'une huile utilisée sans autre purification pour les étapes ultérieures.

### Préparation de l'éthoxycarbonyl-3 difluoro-7,8 méthyl-1 oxo-4 tétrahydro-1,2,3,4 benzo[b]naphtyridine-1,8 :

A une solution de 26,6 g d'éthylate de sodium portée à reflux dans 900 cm³ d'éthanol absolu on ajoute en 80 minutes, une solution de 94 g d'éthoxycarbonyl-3 difluoro-6,7 (N-méthyl N-β éthoxy-carbonyléthyl) amino-2 quinoléine dans 300 cm3 d'éthanol absolu. La suspension obtenue, toujours à reflux, est agitée 15 minutes supplémentaires. On verse ensuite, en 30 minutes, 38 cm³ d'acide acétique glacial. Le mélange réactionnel est agité 15 minutes supplémentaires puis on verse en 45 minutes, toujours à reflux, 500 cm³ d'eau. La suspension obtenue est refroidie à 20°C environ. Le précipité est essoré à 20°C environ et lavé par 2 fois 300 cm³ d'eau. Le produit humide est séché sous pression réduite (20 kPa) à environ 60°C. On isole 71,5 g d'éthoxycarbonyl-3 difluoro-7,8 méthyl-1 oxo-4 tétrahydro-1,2,3,4 benzo[b]naphtyridine-1,8 sous forme d'un solide jaune fondant à 188°C.

### Préparation de l'éthoxycarbonyl-3 difluoro-7,8 méthyl-1 oxo-4 dihydro-1,4 benzo[b]naphtyridine-1,8 :

A une suspension de 71 g d'éthoxycarbonyl-3 difluoro-7,8 méthyl-1 oxo-4 tétrahydro-1,2,3,4 benzo[b]naphtyridine-1,8 dans 1000 cm³ d'éthanol, on ajoute sous agitation à environ 20°C une solution de 3,78 g d'iodure de potassium dans 20 cm³ d'eau. La suspension est chauffée à 77°C et on ajoute à cette température, en 60 minutes, 30 cm³ d'eau oxygénée à 33 % en poids. Le mélange réactionnel est maintenu à reflux 30 minutes supplémentaires puis est refroidi à environ 20°C. A cette température on verse, en 5 minutes, une solution de 11,4 g de thiosulfate de sodium dans 50 cm³ d'eau. Le précipité obtenu est essoré à 20°C environ et lavé par 2 fois 300 cm³ d'eau. Le produit humide obtenu est séché sous pression réduite (20 kPa) à environ 60°C. On isole 73 g d'éthoxycarbonyl-3 difluoro-7,8 méthyl-1 oxo-4 dihydro-1,4 benzo[b]naphtyridine-1,8 sous forme d'un solide blanc fondant à une température supérieure à 270°C.

### Exemple d'utilisation 3

### Préparation de l'éthoxycarbonyl-3 difluoro-6,7 (N-éthyl N-β éthoxycarbonyléthyl) amino-2 quinoléïne :

A une solution de 10 g de chloro-2 éthoxycarbonyl-3 difluoro-6,7 quinoléïne préparée comme décrit à l'exemple 1 et 9,7 g de N-éthyl, N-β éthoxycarbonyléthyl amine dans 120 cm³ de toluène, on ajoute 7,8 g de carbonate de sodium. La suspension obtenue est chauffée à environ 90°C puis agitée 4 heures à cette température. Le mélange ractionnel est ensuite refroidi à environ 20°C puis lavé par 3 fois 100 cm³ d'eau. La phase organique est concentrée à sec sous pression réduite (20 kPa) à environ 50°C. On obtient 13 g d'éthoxycarbonyl-3 difluoro-6,7 (N-éthyl N-β éthoxycarbonyléthyl) amino-2 quinoléïne sous forme d'une huile qui est utilisée sans autre purification pour les étapes ultérieures.

### Préparation de l'éthoxycarbonyl-3 difluoro-7,8 éthyl-1 oxo-4 tétrahydro-1,2,3,4 benzo[b]naphtyridine-1,8 :

A une solution de 16,1 g d'éthylate de sodium porté à reflux dans 600 cm³ d'éthanol absolu on verse, en 60 minutes, une solution de 68 g d'éthoxycarbonyl-3 difluoro-6,7 (N-éthyl N-β éthoxycarbonyléthyl) amino-2 quinoléine dans 200 cm³ d'éthanol absolu. La suspension obtenue, toujours, à reflux, est agitée 60 minutes supplémentaires. On verse, ensuite, en 30 minutes, 20 cm³ d'acide acétique glacial. Le mélange réactionnel est agité 15 minutes supplémentaires puis on verse en 45 minutes, toujours à reflux, 400 cm³ d'eau. La suspension obtenue est refroidie à 20°C environ. Le précipité obtenu est essoré à 20°C environ et lavé par deux fois 200 cm³ d'eau. Le produit humide est séché sous pression réduite (20 kPa) à environ 50°C. On isole 52,4 g d'éthoxycarbonyl-3 difluoro-7,8 éthyl-1 oxo-4 tétrahydro-1,2,3,4 benzo[b]naphtyridine-1,8 sous forme d'un solide jaune d'or fondant à 152°C.

### Préparation de l'éthoxycarbonyl-3 difluoro-7,8 éthyl-1 oxo-4 dihydro-1,4 benzo[b]naphtyridine-1,8 :

A une suspension de 33 g d'éthoxycarbonyl-3 difluoro-7,8 éthyl-1 oxo-4 tétrahydro-1,2,3,4 benzo[b]naphtyridine-1,8 dans 1000 cm³ d'éthanol, on ajoute sous agitation à environ 20°C une solution de 1,7 g d'iodure de potassium dans 10 cm³ d'eau. La suspension est chauffée à 77°C et on verse à cette température, en 30 minutes, 12,7 cm³ d'eau oxygénée à 33 % en poids. Le mélange réactionnel est maintenu à reflux 30 minutes supplémentaires puis est refroidi à environ 20°C. A cette température on verse en 5 minutes une solution de 6 g de thiosulfate de sodium dans 20 cm³ d'eau. Le précipité obtenu est essoré à 20°C environ et lavé par deux fois 150 cm³ d'eau. Le produit humide obtenu est séché sous pression réduite (20 kPa) à environ 50°C. On isole 28,7 g d'éthoxycarbonyle-3 difluoro-7,8 éthyl-1 oxo-4 dihydro-1,4 benzo[b]naphtyridine-1,8 sous forme d'une solide jaune clair fondant à 270°C.

### Exemple d'utilisation 4

Préparation de l'éthoxycarbonyl-3 difluoro-6,7 (N-cyclopropyl N-β éthoxycarbonyléthyl) amino-2 quinoléïne :

A une solution de 3,48 g de chloro-2 éthoxycarbonyl-3 difluoro-6,7 quinoléine préparée comme décrit à l'exemple 1 et 3 g de N-cyclopropyl N-β éthoxycarbonyléthyl amine dans 10 cm³ de toluène, on ajoute 3 g de carbonate de sodium. La suspension obtenue est chauffée à reflux puis agitée 15 heures à cette temprérature. Le mélange réactionnel est ensuite refroidi à environ 20°C puis on ajoute 30 cm³ d'eau et 4,5 cm³ d'acide acétique. Après décantation, le mélange réactionnel est lavé par 2 fois 10 cm³ d'eau. La phase organique est concentrée à sec sous pression réduite (20 kPa) à environ 50°C. On obtient 3,3 g d'éthoxycarbonyl-3 difluoro-6,7 (N-cyclopropyl N-β éthoxycarbonyléthyl) amino-2 quinoléïne brute sous forme d'une huile qui est utilisée sans autre purification pour l'étape ultérieure.

### Préparation de l'éthoxycarbonyl-3 difluoro-7,8 cyclopropyl-1 oxo-4 tétrahydro-1,2,3,4 benzo[b]naphtyridine-1,8 :

A une solution de 1,6 g d'éthylate de sodium porté à reflux dans 40 cm³ d'éthanol absolu on ajoute, en 60 minutes, une solution d'éthoxycarbonyl-3 difluoro-6,7 (N-cyclopropyl N-β éthoxycarbo-nyléthyl) amino-2 quinoléïne dans 20 cm³ d'éthanol absolu. La solution obtenue est agitée 60 minutes supplémentaires, à reflux. On verse ensuite, en 10 minutes, 2,6 cm³ d'acide acétique glacial. Le mélange réactionnel est agité 15 minutes supplémentaires puis on verse en 5 minutes, toujours à reflux, 26 cm³ d'eau. La suspension obtenue est refroidie à 20°C environ. Le précipité est essoré à 20°C environ et lavé par deux fois 10 cm³ d'eau. Le produit humide est séché sous pression réduite (20 kPa) à environ 60°C. on isole 1,25 g d'éthoxycarbonyl-3 difluoro-7,8 cyclopropyl-1 oxo-4 tétrahydro-1,2,3,4 benzo[b]naphtyridine-1,8 brute sous forme d'un solide jaune fondant à 172°C.

### Préparation de l'éthoxycarbonyl-3 difluoro-7,8 cyclopropyl-1 oxo-4 dihydro-1,4 benzo[b]naphtyridine-1,8 :

A une suspension de 1 g d'éthoxycarbonyl-3 difluoro-7,8 cyclopropyl-1 oxo-4 tétrahydro-1,2,3,4 benzo[b]naphtyridine-1,8 dans 14 cm³ d'éthanol, on ajoute sous agitation à environ 20°C une solution de 0,053 g d'iodure de potassium dans 0,5 cm³ d'eau. La suspension est chauffée à 77°C et on verse à cette température, en 5 minutes, 0,5 cm³ d'eau oxygénée à 33 % en poids. Le mélange réactionnel est maintenu à reflux 60 minutes supplémentaires puis refroidi à environ 20°C. A cette température on verse en 5 minutes, 1,06 cm³ d'une solution 1N de thiosulfate de sodium. Le précipité obtenu est essoré à 20°C environ et lavé par 2 fois 10 cm³ d'eau. Le produit humide obtenu est séché sous pression réduite (20 kPa) à environ 60°C. On isole 0,7 g d'éthoxycarbonyl-3 difluoro-7,8 cyclopropyl-1 oxo-4 dihydro-1,4 benzo[b]naphtyridine-1,8 brute sous forme d'un solide blanc ocre fondant à 210°C.

### Exemple d'utilisation 5

### L'éthoxycarbonyl-3 difluoro-6,7 (N-méthyl N-β-cyanoéthyl amino)-2 quinoléine est préparée de la manière suivante :

A une solution de 16,3 g de chloro-2 éthoxycarbonyl-3 difluoro-6,7 quinoléine préparée comme décrit à l'exemple 1 et 10 g de N-méthyl N-β-cyanoéthyl amine dans 160 cm³ de toluène, on ajoute 19,08 g de carbonate de sodium. La suspension obtenue est chauffée à reflux puis agitée 4 heures à cette température. Le mélange réactionnel est ensuite refroidi à environ 20°C puis lavé par 3 fois 50 cm³ d'eau. La phase organique est concentrée à sec sous pression réduite (20 kPa) à environ 50°C. On obtient 19,17 g d'éthoxycarbonyl-3 difluoro-6,7 (N-méthyl N-β-cyanoéthyl amino)-2 quinoléine sous forme d'une huile qui est utilisée sans autre purification pour les étapes ultérieures.

La cyano-3 difluoro-7,8 méthyl-1 oxo-4 tétrahydro-1,2,3,4 benzo[b]naphtyridine-1,8 est préparée de la manière suivante :

A une solution de 8,74 g de terbutylate de potassium dans 200 cm³ de tétrahydrofuranne refroidi à -10°C on verse, en 60 minutes, une solution de 19,17 g d'éthoxycarbonyl-3 difluoro-6,7 (N-méthyl N-β-cyanoéthyl amino)-2 quinoléine dans 50 cm³ de tétrahydrofuranne. La suspension obtenue est agitée toujours à -10°C durant 30 minutes supplémentaires. On verse ensuite 4 cm³ d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 2 fois par 50 cm3 d'eau, puis séché sous pression réduite (20 kPa). On isole 16,1 g de cyano-3 difluoro-7,8 méthyl-1 oxo-4 tétrahydro-1,2,3,4 benzo[b]naphtyridine-1,8 sous forme d'un solide jaune d'or fondant à 144°C.

### La cyano-3 difluoro-7,8 méthyl-1 oxo-4 dihydro-1,4 benzo[b]naphtyridine-1,8 est préparée de la manière suivante :

A une suspension de 8,6 g de cyano-3 difluoro-7,8 méthyl-1 oxo-4 tétrahydro-1,2,3,4 benzo[b]naphtyridine-1,8 dans 350 cm³ d'éthanol, on ajoute sous agitation à environ 20°C une solution de 0,47 g d'iodure de potassium dans 5 cm³ d'eau. La suspension est chauffée à 77°C et additionnée à cette température, en 10 minutes, de 4 cm³ d'eau oxygénée à 33 % en poids. Le mélange réactionnel est maintenu à reflux 30 minutes supplémentaires puis est refroidi à environ 20°C. A cette température on ajoute, en 5 minutes, 10 cm³ d'une solution de thiosulfate de sodium 1N. Le précipité obtenu est essoré à 20°C environ et lavé par deux fois 20 cm³ d'eau. Le produit humide obtenu est séché sous pression réduite (20 kPa) à environ 50°C. On isole 8 g de cyano-3 difluoro-7,8 méthyl-1 oxo-4 dihydro-1,4 benzo[b]naphtyridine-1,8 sous forme d'un solide jaune clair fondant à 380°C.

### La cyano-3 fluoro-7 méthyl-1 (méthyl-4 pipérazinyl-1)-8 oxo-4 dihydro-1,4 benzo[b]naphtyridine-1,8 est préparée de la manière suivante:

Une suspension de 2,1 g de cyano-3 difluoro-7,8 méthyl-1 oxo-4 dihydro-1,4 benzo[b]naphtyridine-1,8 dans 100 cm³ de diméthylsulfoxyde est chauffée à 80°C en présence de 2 cm³ de N-méthyl pipérazine. Le mélange réactionnel est maintenu à cette température durant 8 heures. La solution obtenue est refroidie à température ambiante et agitée à cette température durant 15 heures. Le précipité formé est filtré, lavé par 3 fois 20 cm³ d'eau, et séché sous vide (20 kPa) à 50°C. On obtient 2,6 g de cyano-3 fluoro-7 méthyl-1 (méthyl-4 pipérazinyl-1)-8 oxo-4 benzo[b]naphtyridine-1,8 sous forme d'un précipité jaune fondant à 335°C.

### L'acide fluoro-7 méthyl-1 (méthyl-4 pipérazinyl-1)-8 oxo-4 dihydro-1,4 benzo[b]naphtyridine-1,8 carboxylique-3 est préparé de la manière suivante :

Une suspension de 2 g de cyano-3 fluoro-7 méthyl-1 (méthyl-4 pipérazinyl-1)-8 oxo-4 dihydro-1,4 benzo[b]naphtyridine-1,8 est chauffé à reflux dans 40 cm³ d'acide chlorhydrique 12N. Le mélange réactionnel est maintenu à cette température durant 15 heures. La solution obtenue est refroidie à température ambiante. Le produit qui cristallise est filtré, lavé à l'eau jusqu'à neutralité, et séché sous pression réduite (20 kPa) à 50°C. On obtient 1,5 g d'acide fluoro-7 méthyl-1 (méthyl-4 pipérazinyl-1)-8 oxo-4 dihydro-1,4 benzo[b]naphtyridine-1,8 carboxylique-3 monochlorhydrate sous forme de cristaux jaunes fondant à 290°C (décomposition).

### Exemple d'utilisation 6

### L'éthoxycarbonyl-3 difluoro-6,7 [N-méthyl N-β (N',N'-diméthylaminocarbonyléthyl) amino]-2 quinoléine est préparée de la manière suivante :

A une solution de 26 g de chloro-2 éthoxycarbonyl-3 difluoro-6,7 quinoléine préparée comme décrit à l'exemple 1 et 25 g de N-méthyl N-β (N',N'-diméthylaminocarbonyléthyl) amine dans 300 cm³ de toluène, on ajoute 31 g de carbonate de sodium. La suspension obtenue est chauffée à reflux puis agitée 2 heures 30 minutes à cette température. Le mélange réactionnel est ensuite refroidi à environ 20°C puis lavé par 3 fois 100 cm³ d'eau. La phase organique est concentrée à sec sous pression réduite (20 kPa) à environ 50°C. On obtient 35 g d'éthoxycarbonyl-3 difluoro-6,7 [N-méthyl N-β (N',N'-diméthylaminocarbonyl éthyl) amino]-2 quinoléine sous forme d'une huile qui est utilisée sans autre purification pour les étapes ultérieures.

### La N,N-diméthyl difluoro-7,8 méthyl-1 oxo-4 tétrahydro-1,2,3,4 benzo[b]naphtyridine-1,8 carboxamide-3 est préparée de la manière suivante :

A une solution de 15,7 g de terbutylate de potassium dans 150 cm³ de tétrahydrofuranne refroidi à 0°C on ajoute, en 75 minutes, une solution de 35 g d'éthoxycarbonyl-3 difluoro-6,7 N-méthyl N-β (N',N'-diméthylaminocarbonyl éthyl) amino-2 quinoléine dans 150 cm³ de tétrahydrofuranne. La suspension obtenue est ensuite agitée à 0°C durant 30 minutes supplémentaires puis on ajoute 8 cm³ d'acide acétique glacial. Le tétrahydrofuranne est évaporé sous pression réduite (20 kPa). Le mélange brut réactionnel est repris par 200 cm³ d'un mélange hydroalcoolique éthanol/eau (70/30 vol/vol). Le précipité obtenu est filtré, lavé 3 fois par 100 cm³ d'eau, puis séché sous vide (20 kPa). On isole 25 g de N,N-diméthyl difluoro-7,8 méthyl-1 oxo-4 tétrahydro-1,2,3,4 benzo[b]naphtyridine-1,8 carboxamide-3 sous forme d'un solide jaune citron fondant à 206°C .

### La N,N-diméthyl difluoro-7,8 méthyl-1 oxo-4 dihydro-1,4 benzo[b] naphtyridine-1,8 carboxamide-3 est préparée de la manière suivante :

A une suspension de 25 g de N,N-diméthyl difluoro-7,8 méthyl-1 oxo-4 tétrahydro-1,2,3,4 benzo[b]naphtyridine-1,8 carboxamide-3 dans 1000 cm³ d'éthanol, on ajoute sous agitation à environ 20°C une solution de 1,35 g d'iodure de potassium dans 10 cm³ d'eau. La suspension est chauffée à 77°C et on verse à cette température, en 20 minutes, 25 cm³ d'eau oxygénée à 33 % en poids. Le mélange réactionnel est maintenu à reflux 1 heure 30 minutes supplémentaire puis est refroidi à environ 20°C. A cette température on coule,en 5 minutes, 30 cm³ d'une solution de thiosulfate de sodium 1N. Le précipité obtenu est essoré à 20°C environ et lavé par deux fois 60 cm³ d'eau. Le produit humide obtenu est séché sous pression réduite (20 kPa) à environ 50°C. On isole 19,5 g de N,N-diméthyl difluoro-7,8 méthyl-1 oxo-4 dihydro-1,4 benzo[b]naphtyridine-1,8 carboxamide-3 sous forme d'un solide jaune clair fondant à 324°C.

Une suspension de 2,96 g de difluoro-7,8 N,N-diméthyl oxo-4 méthyl-1 dihydro-1,4 benzo[b]naphtyridine-1,8 carboxamide-3, de 1,12 g de méthyl-1 pipérazine et de 1,55 g de carbonate de potassium dans 100 cm³ de diméthylsulfoxyde est chauffée 5 heures, sous agitation, à environ 80°C. Après refroidissement à environ 20°C, le mélange réactionnel est additionné de 100 cm³ d'eau ; l'insoluble est essoré, lavé par 2 fois 30 cm³ d'eau et 2 fois 30 cm³ d'éthanol.

On obtient 2,3 g de N,N diméthyl fluoro-7 méthyl-1 (méthyl-4 pipérazinyl-1)-8 oxo-4 dihydro-1,4 benzo[b]naphtyridine-1,8 carboxamide-3 sous forme d'un solide jaune se décomposant à 275°C.

Une solution de 0,5 g de N,N-diméthyl fluoro-7 méthyl-1 (méthyl-4 pipérazinyl-1)-8 oxo-4 dihydro-1,4 benzo[b]naphtyridine-1,8 carboxamide-3 dans 10 cm³ d'acide chlorhydrique aqueux 6N est chauffée, sous agitation à environ 95°C, pendant 5 heures. Après refroidissement à environ 20°C, l'insoluble est essoré, lavé par 3 fois 20 cm³ d'eau et 2 fois 10 cm³ d'éthanol.

Le produit obtenu est mis en suspension dans 30 cm³ d'eau ; on ajoute 0,6 cm³ de potasse aqueuse N et agite 1 heure à environ 20°C. L'insoluble est essoré, lavé par 2 fois 20 cm³ d'eau et 2 fois 10 cm³ d'éthanol. Après recristallisation dans 15 cm³ diméthyformamide, on obtient 0,15 g d'acide fluoro-7 méthyl-1 (méthyl-4 pipérazinyl-1)-8 oxo-4 dihydro-1,4 benzo[b]naphtyridine-1,8 carboxylique-3 sous forme d'un solide jaune se décomposant à 354°C.

### Exemple d'utilisation 7

### L'éthoxycarbonyl-3 difluoro-6,7 (N-méthyl N-β aminocarbonyléthyl amino)-2 quinoléine est préparée de la manière suivante :

A une solution de 4 g de chloro-2 éthoxycarbonyl-3 difluoro-6,7 quinoléine préparée comme décrit à l'exemple 1 et 3 g de N-méthyl N-β-aminocarbonyléthyl amine dans 40 cm³ de toluène, on ajoute 4,4 g de carbonate de sodium. La suspension obtenue est chauffée à reflux puis agitée 2 heures 30 minutes à cette température. Le mélange réactionnel est ensuite refroidi à environ 20°C puis lavé par 3 fois 25 cm³ d'eau. La phase organique est concentrée à sec sous pression réduite (20 kPa) à environ 50°C. On obtient 4,7 g d'éthoxycarbonyl-3 difluoro-6,7 (N-méthyl N-β-ami-nocarbonyléthyl amino)-2 quinoléine sous forme d'une huile qui est utilisée sans autre purification pour les étapes ultérieures.

### La carboxamide-3 difluoro-7,8 méthyl-1 oxo-4 tétrahydro-1,2,3,4 benzo[b]naphtyridine-1,8 est préparée de la manière suivante :

A une solution de 1,8 g de terbutylate de potassium dans 50 cm³ de tétrahydrofuranne refroidi à 0°C on ajoute, en 30 minutes, une solution de 4,23 g d'éthoxycarbonyl-3 difluoro-6,7 (N-méthyl N-β- aminocarbonyléthyl amino)-2 quinoléine dans 20 cm³ de tétrahydrofuranne. La suspension obtenue est ensuite agitée à 0°C durant 30 minutes supplémentaires puis on verse 2 cm³ d'acide acétique glacial. Le tétrahydrofuranne est évaporé sous pression réduite (20 kPa). Le brut réactionnel est repris par 10 cm³ d'un mélange hydroalcoolique éthanol/eau ( 70/30 vol/vol). Le précipité obtenu est filtré, lavé 3 fois par 10 cm³ d'eau, puis séché sous pression réduite (20 kPa). On isole 1,6 g de carboxamide-3 difluoro-7,8 méthyl-1 oxo-4 tétrahydro-1,2,3,4 benzo[b]naphtyridine-1,8 sous forme d'un solide jaune fondant à 182°C.

### La carboxamide-3 difluoro-7,8 méthyl-1 oxo-4 dihydro-1,4 benzo[b] naphtyridine-1,8 est préparée de la manière suivante :

A une suspension de 1,3 g de carboxamide-3 difluoro-7,8 méthyl-1 oxo-4 tétrahydro-1,2,3,4 benzo[b]naphtyridine-1,8 dans 25 cm³ d'éthanol, on ajoute sous agitation à environ 20°C une solution de 0,1 g d'iodure de potassium dans 1 cm³ d'eau. La suspension est chauffée à 77°C et additionnée à cette température, en 5 minutes, de 1,5 cm³ d'eau oxygénée à 33 % en poids . Le mélange réactionnel est maintenu à reflux 1 heure 30 minutes supplémentaire puis est refroidi à environ 20°C. A cette température on ajoute 1 cm³ d'une solution de thiosulfate de sodium 1N. Le précipité obtenu est essoré à 20°C environ et lavé par deux fois 5 cm³ d'eau. Le produit humide obtenu est séché sous pression réduite (20 kPa) à environ 50°C. On isole 1,1 g de carboxamide-3 difluoro-7,8 méthyl-1 oxo-4 dihydro-1,4 benzo[b]naphtyridine-1,8 sous forme d'un solide orangé fondant à 318°C .

Une suspension de 1,3 g de difluoro-7,8 méthyl-1 oxo-4 dihydro-1,4 benzo[b]naphtyridine-18 carboxamide-3, de 0,54 g de méthyl-1 pipérazine et de 0,75 g de carbonate de potassium dans 25 cm³ de diméthylsulfoxyde est chauffée à environ 80°C pendant 6 heures. Après refroidissement à une température voisine de 20°C, le mélange réactionnel est additionné de 100 cm³ d'eau. L'insoluble est essoré, lavé par 2 fois 20 cm³ d'eau et 2 fois 20 cm³ d'éthanol.

Le produit obtenu est chromatographié sur 20 g de gel de silice (0,063-0,200 mm) en suspension dans un mélange de dichlorométhane à 10 % de méthanol. On élimine de impuretés réactionnelles par élution avec 500 cm³ de ce mélange de solvants. Le produit attendu est ensuite élué par 500 cm³ du même mélange de solvants. Après concentration à sec, sous pression réduite (20 kPa) à envion 40°C, le résidu solide est recristallisé dans 25 cm³ de diméthylformamide, essoré et lavé par 2 fois 30 cm³ d'éthanol à environ 70°C.

On obtient 0,6 g de fluoro-7 méthyl-1 (méthyl-4 pipérazine-1)-8 oxo-4 dihydro-1,4 benzo[b]naphtyridine-1,8 carboxamide-3 sous forme d'un solide jaune se décomposant à 265°C.

L'acide fluoro-7 (méthyl-4 pipérazinyl-1)-8 méthyl-1 oxo-4 dihydro-1,4 benzo[b]naphtyridine-1,8 carboxylique-3 est préparé dans les conditions de l'exemple d'utilisation 2, mais à partir de 0,3 g de fluoro-7 méthyl-1 (méthyl-4 pipérazinyl-1)-8 oxo-4 dihydro-1,4 benzo[b]naphtyridine-1,8 carboxamide-3. Après refroidissement à environ 20°C, le mélange réactionnel est additionné de 50 cm³ d'eau ; l'insoluble est essoré et lavé par 2 fois 10 cm³ d'eau.

Le produit obtenu est mis en suspension dans 20 cm³ d'eau, additionné de 0,4 cm³ d'une solution de potasse aqueuse N et agité pendant 1 heure à environ 20°C. L'insoluble est essoré, lavé par 3 fois 10 cm³ d'eau, 2 fois 10 cm³ d'éthanol et recristallisé dans 20 cm³ de diméthylformamide.

On obtient 0,17 g d'acide fluoro-7 méthyl-1 (méthyl-4 pipérazinyl-1)-8 oxo-4 dihydro-1,4 benzo[b]naphtyridine-1,8 carboxylique-3, sous forme d'un solide jaune se décomposant à 354°C.

## Revendications

1. Un procédé de préparation de fluoro-6 halogéno-2 quinoléine de formule générale : 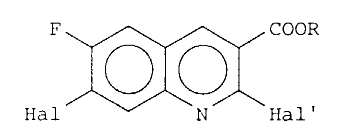 dans laquelle R est un atome d'hydrogène ou un radical alcoyle, Hal est un atome d'halogène et Hal' est un atome de chlore ou de brome, caractérisé en ce que l'on fait agir un agent d'halogénation choisi parmi le trichlorure de phosphore, le pentachlorure de phosphore, le chlorure de thionyle, le chlorure de sulfuryle, le dichlorosulfure, le chlorure stanneux, le chlorure cuivreux, le trichlorure de titane, le chlorure ferreux, le chlorure de chrome^{II}, l'hydrochloro triphénylphosphorane, le dichloro triphénylphosphorane ou le chlore, ou choisi parmi le tribromure de phosphore, l'hydrobromo triphénylphosphorane ou le dibromo triphénylphosphorane, sur l'hydroxy-1 quinolone de formule générale: 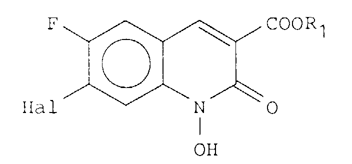 dans laquelle Hal est défini comme ci-dessus et R₁ est défini comme R à l'exception de représenter un atome d'hydrogène, puis éventuellement libère la fonction acide si l'on veut obtenir une fluoro-6 halogéno-2 quinoléine pour laquelle R est un atome d'hydrogène.

2. Un procédé de préparation de fluoro-6 halogéno-2 quinoléine selon la revendication 1, caractérisé en ce que le symbole Hal est le chlore ou le fluor.

3. Un procédé de préparation de fluoro-6 halogéno-2 quinoléine selon la revendication 1, caractérisé en ce que l'hydroxy-1 quinolone de départ est préparée par cyclisation d'un dérivé nitré de formule générale : 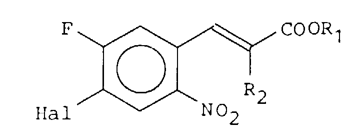 dans laquelle Hal et R₁ sont définis comme dans la revendication 1, et R₂ est défini comme R₁ ou représente un radical carbamoyle ou cyano, par hydrogénation catalytique en milieu acide.

4. Utilisation d'un procédé selon l'une des revendications 1 à 3, pour l'obtention d'un dérivé de la benzo[b]naphtyridine-1,8 de formule générale : 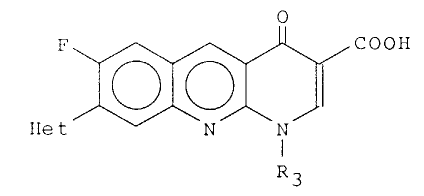 dans laquelle soit R₃ est un radical alcoyle, fluoroalcoyle, cycloalcoyle contenant 3 à 6 atomes de carbone, alcoyloxy ou alcoylamino et Het est un radical hétérocyclyle azoté, soit R₃ est un atome d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino,et Het est un radical azétidinyl-1 substitué [en position -3 par un radical R₄ qui peut être un atome d'hydrogène ou un radical hydroxy, amino, alcoylamino dont la partie alcoyle est éventuellement substituée par un radical amino ou hydroxy ou peut représenter un radical dialcoylamino dont les parties alcoyle peuvent éventuellement former avec l'atome d'azote auquel elles sont rattachées, un hétérocycle à 5 ou 6 chaînons contenant éventuellement un autre hétéroatome choisi parmi l'azote, l'oxygène ou le soufre, ou peut représenter un radical cycloalcoylamino contenant 3 à 6 chaînons, ou un radical alcanoylamino, N-alcoyl N-alcanoyl amino ou aminoalcoylphénylamino, et substitué en positions -2 et -3 par des radicaux R₅ et R₆ identiques ou différents qui représentent des atomes d'hydrogène, des radicaux alcoyle, alcènyle contenant 2 à 4 atomes de carbone, phényle, phényle substitué par un atome d'halogène, ou par un radical alcoyle, alcoyloxy, hydroxy, nitro, amino, alcoylamino, dialcoylamino ou halogénoalcoyle, ou bien substitué en position -2 par des radicaux R₅ et R₆ qui représentent des radicaux alcoyle], les radicaux alcoyle et alcanoyle cités ci-dessus étant droits ou ramifiés et contennant 1 à 4 atomes de carbone.

5. Utilisation d'un procédé selon l'une des revendications 1 à 3, pour l'obtention d'un dérivé de la benzonaphtyridine de formule générale : 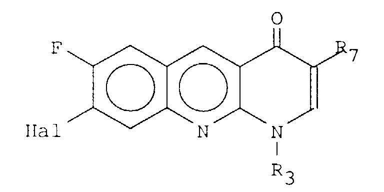 dans laquelle Hal est défini comme dans la revendication 1, R₃ représente un atone d'hydrogène ou un radical alcoyle, fluoroalcoyle, carboxyalcoyle, cycloalcoyle contenant 3 à 6 atomes de carbone, fluorophényle, difluorophényle, alcoyloxy ou alcoylamino et R₇ est un radical alcoyloxycarbonyle, cyano, carbamoyle, alcoylcarbamoyle, benzylcarbamoyle, hydroxyéthylcarbamoyle, dialcoylaminoéthylcarbamoyle ou dialcoylcarbamoyle dont les parties alcoyle peuvent éventuellement former avec l'atome d'azote auquel elles sont rattachées, un hétérocycle à 5 ou 6 chaînons contenant éventuellement un autre hétéroatome choisi parmi l'oxygène, le soufre ou l'azote et éventuellement substitué sur l'azote par un radical alcoyle, les radicaux alcoyle étant droits ou ramifiés et contenant 1 à 4 atomes de carbone.

## Patentansprüche

1. Verfahren zur Herstellung von 6-Fluor-2-halogen-chinolin der allgemeinen Formel 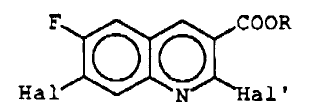 in der R ein Wasserstoffatom oder einen Rest Alkyl darstellt, Hal ein Halogenatom ist und Hal' ein Atom von Chlor oder Brom bedeutet, dadurch gekennzeichnet, daß man ein Halogenierungsmittel, ausgewählt unter Phosphortrichlorid, Phosphorpentachlorid, Thionylchlorid, Sulfurylchlorid, Schwefeldichlorid, Zinn(II)-chlorid, Kupter(I)-chlorid, Titantrichlorid, Eisen(II)-chlorid, Chrom(II)-chlorid, Hydrochlor-triphenylphosphoran, Dichlor-triphenylphosphoran und Chlor, oder ausgewählt unter Phosphortribromid, Hydrobrom-triphenylphosphoran oder Dibrom-triphenylphosphoran, mit 1-Hydroxy-chinolon der allgemeinen Formel 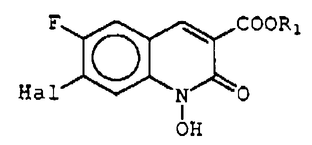 zur Reaktion bringt, in der Hal wie oben und R₁ wie R definiert ist, mit der Ausnahme, ein wasserstoffatom darzustellen, und man anschließend gegebenenfalls die Säurefunktion freisetzt, wenn man wünscht, ein 6-Fluor-2-halogen-chinolin zu erhalten, worin R ein Wasserstoffatom ist.

2. Verfahren zur Herstellung von 6-Fluor-2-halogen-chinolin nach Anspruch 1, dadurch gekennzeichnet, daß das Symbol Hal Chlor oder Fluor ist.

3. Verfahren zur Herstellung von 6-Fluor-2-halogen-chinolin nach Anspruch 1, dadurch gekennzeichnet, daß das eingesetzte 1-Hydroxy-chinolon durch Cyclisierung eines Nitroderivates der allgemeinen Formel 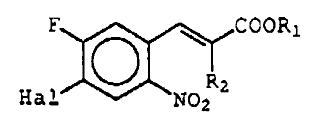 in der Hal und R₁ wie in Anspruch 1 definiert sind und R₂ wie R₁ definiert ist oder einen Rest Carbamoyl oder Cyano bedeutet, mittels katalytischer Hydrierung im sauren Medium hergestellt wird.

4. Anwendung eines Verfahrens nach einem der Ansprüche 1 bis 3 zur Herstellung eines Derivates von 1,8-Benzo[b]-naphthyridin der allgemeinen Formel 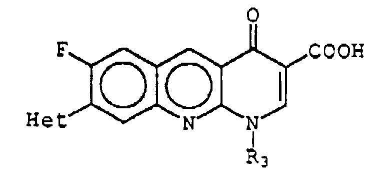 in der entweder
R₃ einen Rest Alkyl, Fluoralkyl, Cycloalkyl mit 3 bis 6 Kohlenstoffatomen, Alkyloxy oder Alkylamino darstellt und Het den Rest eines Stickstoff-Heterocyclus bedeutet, oder
R₃ ein Wasserstoffatom oder einen Rest Alkyl, Fluoralkyl, Carboxyalkyl, Cycloalkyl mit 3 bis 6 Kohlenstoffatomen, Fluorphenyl, Difluorphenyl, Alkyloxy oder Alkylamino darstellt und Het einen substituierten Rest 1-Azetidinyl bedeutet [substituiert in Position -3 durch einen Rest R₄, der ein Wasserstoffatom oder ein Rest Hydroxy, Amino, Alkylamino, dessen Alkylteil gegebenenfalls substituiert ist durch einen Rest Amino oder Hydroxy, sein kann oder auch einen Rest Dialkylamino darstellen kann, dessen Alkylteile gegebenenfalls mit dem Stickstoffatom, an das sie gebunden sind, einen Heterocyclus mit 5 oder 6 Ringgliedern bilden können, der gegebenenfalls ein anderes Heteroatom enthalten kann, ausgewählt unter Stickstoff, Sauerstoff und Schwefel, oder auch einen Rest Cycloalkylamino mit 3 bis 6 Ringgliedern, einen Rest Alkanoylamino, N-Alkyl-N-alkanoyl-amino oder Aninoalkylphenylamino bedeuten kann und substituiert in den Positionen -2 und -3 durch gleiche oder verschiedene Reste R₅ und R₆, die Wasserstoffatome, Reste Alkyl, Alkenyl mit 2 bis 4 Kohlenstoffatomen, Phenyl, Phenyl, substituiert durch ein Halogenatom oder durch einen Rest Alkyl, Alkyloxy, Hydroxy, Nitro, Amino, Alkylamino, Dialkylamino oder Halogenalkyl, darstellen, oder auch substituiert in Position -2 durch Reste R₅ und R₆, die Reste Alkyl sind], wobei die oben genannten Reste Alkyl und Alkanoyl gerade oder verzweigt sind und 1 bis 4 Kohlenstoffatome enthalten.

5. Anwendung eines Verfahrens nach einem der Ansprüche 1 bis 3 zur Herstellung eines Derivates von Benzonaphthyridin der allgemeinen Formel 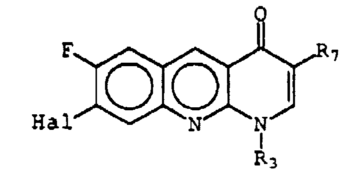 in der Hal wie in Anspruch 1 definiert ist, R₃ ein Wasserstoffatom oder einen Rest Alkyl, Fluoralkyl, Carboxyalkyl, Cycloalkyl mit 3 bis 6 Kohlenstoffatomen, Fluorphenyl, Difluorphenyl, Alkyloxy oder Alkylamino darstellt und R₇ einen Rest Alkyloxycarbonyl, Cyano, Carbamoyl, Alkylcarbamoyl, Benzylcarbamoyl, Hydroxyethylcarbamoyl, Dialkylaminoethylcarbamoyl oder Dialkylcarbamoyl bedeutet, dessen Alkylteile gegebenenfalls zusammen mit dem Stickstoffatom, an das sie gebunden sind, einen Heterocyclus mit 5 oder 6 Ringgliedern bilden können, der gegebenenfalls ein anderes Heteroatom enthalten kann, ausgewählt unter Sauerstoff, Schwefel und Stickstoff, und gegebenenfalls substituiert am Stickstoff durch einen Rest Alkyl, wobei die Reste Alkyl gerade oder verzweigt sind und 1 bis 4 Kohlenstoffatome enthalten.

## Claims

1. A process for the preparation of a 6-fluoro-2-haloquinoline of general formula 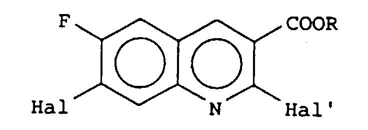 in which R is a hydrogen atom or an alkyl radical, Hal is a halogen atom and Hal' is a chlorine or bromine atom, characterized in that a halogenating agent chosen from phosphorus trichloride, phosphorus pentachloride, thionyl chloride, sulphuryl chloride, sulphur dichloride, stannous chloride, cuprous chloride, titanium trichloride, ferrous chloride, chromium(II) chloride, triphenylphosphine hydrochloride, dichlorotriphenylphosphorane or chlorine, or chosen from phosphorus tribromide, triphenylphosphine hydrobromide or dibromotriphenylphosphorane, is reacted with the 1-hydroxyquinolone of general formula: 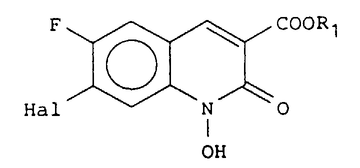 in which Hal is defined as above and R₁ is defined as R with the exception of representing a hydrogen atom, optionally followed by freeing the acidic function if it is desired to obtain a 6-fluoro-2-haloquinoline for which R is a hydrogen atom.

2. A process for the preparation of a 6-fluoro-2-haloquinoline according to claim 1, characterized in that the symbol Hal is chlorine or fluorine.

3. A process for the preparation of a 6-fluoro-2-haloquinoline according to claim 1, characterized in that the starting 1-hydroxyquinolone is prepared by cyclization of a nitro derivative of general formula: 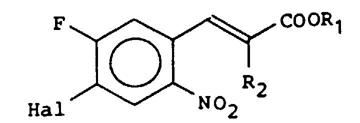 in which Hal and R₁ are defined as in claim 1, and R₂ is defined as R₁ or represents a carbamoyl or cyano radical, by catalytic hydrogenation in acidic medium.

4. Use of a process according to one of claims 1 to 3, in order to obtain a 1,8-benzo[b]naphthyridine derivative of general formula: 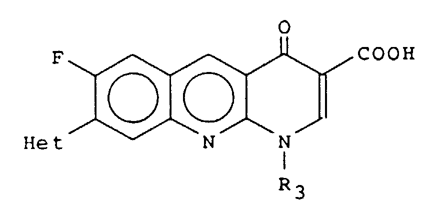 in which either R₃ is an alkyl, fluoroalkyl, cycloalkyl containing 3 to 6 carbon atoms, alkyloxy or alkylamino radical and Het is a nitrogen-containing heterocyclic radical, or R₃ is a hydrogen atom or an alkyl, fluoroalkyl, carboxyalkyl, cycloalkyl containing 3 to 6 carbon atoms, fluorophenyl, difluorophenyl, alkyloxy or alkylamino radical, and Het is a 1-azetidinyl radical which is substituted [in the 3-position with a radical R₄ which may be a hydrogen atom or a hydroxyl, amino or alkylamino radical in which the alkyl part is optionally substituted with an amino or hydroxyl radical or may represent a dialkylamino radical for which the alkyl parts may optionally form, with the nitrogen atom to which they are attached, a 5- or 6-membered heterocycle optionally containing another hetero atom chosen from nitrogen, oxygen or sulphur, or may represent a cycloalkylamino radical containing 3 to 6 members, or an alkanoylamino, N-alkyl-N-alkanoylamino or aminoalkylphenylamino radical, and substituted in the 2- and 3-positions with radicals R₅ and R₆, which may be identical or different and which represent hydrogen atoms, alkyl radicals, alkenyl radicals containing 2 to 4 carbon atoms, phenyl radicals or phenyl radicals substituted with a halogen atom, or with an alkyl, alkyloxy, hydroxyl, nitro, amino, alkylamino, dialkylamino or haloalkyl radical, or alternatively substituted in the 2-position with radicals R₅ and R₆ which represent alkyl radicals], the alkyl and alkanoyl radicals mentioned above being straight or branched and containing 1 to 4 carbon atoms.

5. Use of a process according to one of claims 1 to 3, in order to obtain a benzonaphthyridine derivative of general formula: 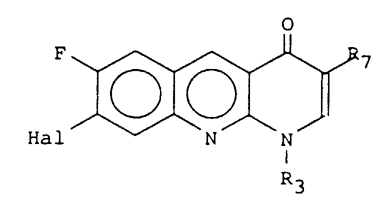 in which Hal is defined as in claim 1, R₃ represents a hydrogen atom or an alkyl, fluoroalkyl, carboxyalkyl, cycloalkyl containing 3 to 6 carbon atoms, fluorophenyl, difluorophenyl, alkyloxy or alkylamino radical and R₇ is an alkyloxycarbonyl, cyano, carbamoyl, alkylcarbamoyl, benzylcarbamoyl, hydroxyethylcarbamoyl, dialkylaminoethylcarbamoyl or dialkylcarbamoyl radical in which the alkyl parts may optionally form, with the nitrogen atom to which they are attached, a 5- or 6-membered heterocycle optionally containing another hetero atom chosen from oxygen, sulphur or nitrogen and optionally substituted on the nitrogen with an alkyl radical, the alkyl radicals being straight or branched and containing 1 to 4 carbon atoms.
